(19)

**Europäisches Patentamt**
**European Patent Office**
**Office européen des brevets**

(11) **EP 4 259 387 B1**

(12) **EUROPEAN PATENT SPECIFICATION**

(45) Date of publication and mention
of the grant of the patent:
**03.12.2025  Bulletin 2025/49**

(51) International Patent Classification (IPC):
**B25J 9/10** *(2006.01)*        **B25J 9/12** *(2006.01)*
**A61B 34/00** *(2016.01)*       **A61M 25/01** *(2006.01)*

(21) Application number: **21904326.2**

(52) Cooperative Patent Classification (CPC):
**B25J 9/104; A61B 1/0052; A61B 34/71;**
**B25J 9/003; B25J 13/02;** A61B 2017/00327;
A61B 2034/301; A61M 25/0136; A61M 25/0147;
A61M 2025/0166

(22) Date of filing: **08.12.2021**

(86) International application number:
**PCT/US2021/062391**

(87) International publication number:
**WO 2022/125655 (16.06.2022 Gazette 2022/24)**

(54) **CABLE DRIVEN PARALLEL MANIPULATOR CONTROL MECHANISM**

KABELBETRIEBENER PARALLELER MANIPULATORSTEUERUNGSMECHANISMUS

MÉCANISME DE COMMANDE DE MANIPULATEUR PARALLÈLE ENTRAÎNÉ PAR CÂBLES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **10.12.2020   US 202063123981 P**

(43) Date of publication of application:
**18.10.2023   Bulletin 2023/42**

(73) Proprietor: **Magellan Biomedical Inc.**
**Richmond Hill, ON L4C5X (CA)**

(72) Inventors:
- **TAVALLAEI, Mohammed Ali**
  **Scarborough, ON M1L 0H4 (CA)**
- **QUADRI, Amaar**
  **Scarborough, ON M1L 0H4 (CA)**
- **WRIGHT, Graham A.**
  **Scarborough, ON M1L 0H4 (CA)**
- **SEWANI, Alykhan**
  **Scarborough, ON M1L 0H4 (CA)**
- **ZHOU, James Jiewen**
  **Richmond Hill, ON L4C5X4 (CA)**
- **MAGNIN, Christopher J.**
  **Richmond Hill, ON L4C5X4 (CA)**

(74) Representative: **St Clair Jones, Gregory Arthur**
**Langley**
**Kilburn & Strode LLP**
**Lacon London**
**84 Theobalds Road**
**London WC1X 8NL (GB)**

(56) References cited:
EP-A1- 3 653 105          US-A1- 2007 113 700
US-A1- 2010 179 540     US-A1- 2019 047 705
US-A1- 2019 374 293     US-A1- 2020 015 832
US-A1- 2020 170 701

# EP 4 259 387 B1

**Description**

CROSS-REFERENCE TO RELATED APPLICATION

The present application claims the benefit of U.S. Provisional Application No. 63/123,981 entitled "CABLE DRIVEN PARALLEL MANIPULATOR CONTROL MECHANISM AND RELATED SYSTEMS AND METHODS", filed December 10, 2020.

TECHNICAL FIELD

This disclosure relates to a mechanism for control and actuation of a device using cables or strings and related systems. Embodiments of this mechanism have applications for steering and tracking of interventional devices for cardiovascular procedures. Embodiments can relate to the control and navigation of interventional devices such as guidewires, catheters, needles, and imaging and ablative devices for cardiac and endovascular interventions. Further embodiments relate to steerable robotics for remote camera operation applications, continuum robotics applications, search and rescue applications, and confined space robotics.

Throughout this disclosure and claims, references to "cables" should be understood to broadly refer to any type of strings, wires, or similar manipulable components made of metal, fabrics, polymers, or crystals, for example.

BACKGROUND

Past approaches for controlling a cable-driven apparatus in medical interventional devices have often involved the use of motorized winches attached to the cables. These winches may utilize position and tension feedback to permit proportional adjustment of cable lengths to achieve the desired position of the end effector in a workspace. These winches may also be used to maintain optimal tensioning of the cables and avoid cable sagging (loss of tension). Typically, cable-driven mechanisms with a certain number of cables require use of an equivalent number of motors to control the end-effector position. For this approach, as the number of cables increases, so does the number of needed actuators, leading to increasing costs and complexity for control. Since all the cables are directly connected to the end effector, there is an inherent interdependence in the interaction of the cables. To achieve a required position, the motors must adjust all the cables to account for the interdependent motion. Also, these systems require a frame encompassing the available workspace to leverage the tension from the cables and allow for steering of the end effector. For example, there may be a cable-driven end effector with a two-dimensional planar workspace driven by four cables. Moving to any given position with one motor retracting cable may require the other three motors to release their corresponding cables. If this is not correctly accounted for, the increase in the cable tension could damage the system or jeopardize its performance and accuracy.

Robotics have been used in cable-driven systems in the past. In general, many robotic devices consist of master/slave systems in which the "master" represents the input, and the "slave" represents the output. In the context of this document, the "master" refers to the method of manipulating the cables, and the "slave" refers to the device being controlled and remotely manipulated. For intuitive control, it is essential that the input motion at the master resembles the output motion at the slave.

For a cable-driven system the cables may leverage an expandable structure as the frame that supports the cables for manipulation of a slave device by actuation of the master input unit. Various applications require the use of multiple cables connected to the slave. The mechanical coupling and interconnection of the cables require proportional adjustment of the cables at the input master unit for proper control and actuation of the slave. In a motorized system, this requirement can be accommodated by automatic proportional control of the motors that adjust the length of each cable at the desired tension. However, for a passive system, a solution is required that can account for the interdependence of these cables and allows for proportional adjustment of the lengths of the cables while reducing the system complexity. An appropriate solution is needed that will permit a passive manual slave arrangement while also possibly permitting simpler solutions for motorized configurations.

Accordingly, there is a desire for an improved apparatus or solution permitting the control and actuation of a cable-driven end effector of a desired device with enhanced effectiveness and convenience and which overcomes limitations of the past.

US 2020/170701 A1, US 2010/179540 A1, and EP 3 653 105 A1 represent relevant prior art. EP 4 173 576 A1 is published after the filing date of the present application and is therefore only relevant under Art. 54(3) EPC.

SUMMARY

The invention is defined by independent claim 1. Dependent claims disclose exemplary embodiments.

Embodiments described or otherwise contemplated herein substantially provide accurate control, actuation, and tensioning of cable-actuated end effectors of devices such as interventional devices. The embodiments disclosed herein relate to apparatuses, systems, and methods that allow for simultaneous actuation of multiple interacting cables to control the position of a device in a two-dimensional plane.

One embodiment relates to a cable driven parallel manipulator control mechanism that provides passive cable control. The control mechanism includes a control member, a motion stage, and a cable actuation mechanism. The control member includes a control surface defining a three-dimensional profile. The motion stage has at least two degrees of freedom (DOF) and is operably coupled to the control member. The cable actuation mechanism is located inferior to the control surface and includes at least one connector mechanism and a plurality of cables. The at least one connector mechanism constrained to contact the control surface. The plurality of cables each have a first end and a second end, wherein the first end is connected to one of the at least one connector mechanism and the second end provides coupled attachment to a device being controlled. Further, translation of the control member along a two-dimensional plane causes the at least one connector to travel perpendicularly to the two-dimensional plane resulting in cable displacements that actuate the device being controlled in proportion to translation of the control member.

One embodiment relates to a device control mechanism that controls a plurality of cables. The device control mechanism includes a control member with a control surface defining a three-dimensional conical recess in the bottom of the control member acting as a cam guide surface. The device control mechanism includes a motion stage operably coupled to the control member. The device control mechanism further includes a plurality of pistons, each including a spring and roller constrained to contact the guide surface from below. The device control mechanism also includes a plurality of cables, each having a first end and a second end, wherein the first end is connected to one of the plurality of pistons and the second end is connected to an end effector being controlled, such as an interventional device. The plurality of cables are maintained in tension and are anchored proximate the first ends and second ends as vertices of corresponding polygon shapes.

In certain embodiments, systems and methods incorporate a control surface (e.g., a three-dimensional conical surface) coupled to a two-degree-of-freedom motion stage to act as a master side for providing input to the system. In various embodiments, one end of each included cable is connected to a piston, which is constrained to contact the control surface (e.g., by utilization of springs). The other end of the cables is coupled to the slave device of interest to be controlled. As the control surface is translated along the two-dimensional plane, the pistons travel perpendicularly to the plane, resulting in cable displacements that actuate the slave end effector, such as the distal tip of a catheter, for example. Such systems and methods allow the user to directly adjust the position of the control surface, which results in a corresponding proportional adjustment of the catheter tip via the attached cables.

Some embodiments include a mechanism for actuation and control of an interventional device in two dimensions via a cable-driven mechanism. The mechanism includes multiple spring-loaded cam followers and a conical control surface acting as the cam. The two-degree-of-freedom translation of the conical cam results in the perpendicular linear motion of the cam followers. With the cables coupled to the cam followers from one end, and to the device of interest at the other end, the motion of the followers results in cable displacements that ultimately lead to the manipulation of the end effector, such as an interventional device. This approach allows for adjustment of the cable lengths with a proportion determined by the cam profile, which allows maintaining a set tension and while avoiding sagging. The resulting system can be an entirely passive mechanism that allows for accurate device position control, position estimation, and haptic feedback. Applications of such a device in interventional surgery and imaging, integration with continuum robots, remote camera operation, search and rescue applications, and confined space robotics are contemplated.

The above summary is not intended to describe each illustrated embodiment or every implementation of the subject matter hereof. The figures and the detailed description that follow more particularly exemplify various embodiments.

BRIEF DESCRIPTION OF THE FIGURES

Subject matter hereof may be more completely understood in consideration of the following detailed description of various embodiments in connection with the accompanying figures, in which:

FIG. 1 is an isometric view of a control mechanism for a steerable interventional medical device, according to an embodiment.
FIG. 2 is a view of the side of the control mechanism of FIG. 1, according to an embodiment.
FIG. 3 is a side cross-section view of the control mechanism of FIG. 1, according to an embodiment.
FIG. 4A is a side cross-section view of the control mechanism of FIG. 1 in an alternate position showing modified steering tension, according to an embodiment.
FIG. 4B is a side cross-section view of the control mechanism of FIG. 1 in an alternate position showing a different modified steering tension, according to an embodiment.
FIG. 5A is an end cross-section view of the control mechanism of FIG. 1 in an alternate position, according to an embodiment.

FIG. 5B is an end cross-section view of the control mechanism of FIG. 1 in an alternate position, according to an embodiment.

FIG. 6 is an isometric view of a control mechanism for a steerable interventional medical device, according to an alternate embodiment.

FIG. 7 is a view of the side of a control mechanism of FIG. 6, according to an embodiment.

FIG. 8 is a side cross-section view of the control mechanism of FIG. 6, according to an embodiment.

FIG. 9A is a side cross-section view of the control mechanism of FIG. 6 in an alternate position showing modified steering tension, according to an embodiment.

FIG. 9B is a side cross-section view of the control mechanism of FIG. 6 in an alternate position showing a different modified steering tension, according to an embodiment.

FIG. 10A is an end cross-section view of the control mechanism of FIG. 6 in an alternate position, according to an embodiment.

FIG. 10B is an end cross-section view of the control mechanism of FIG. 6 in an alternate position, according to an embodiment.

FIGS. 11A-D show example diagrams demonstrating the positioning of a three-dimensional control surface that can be used to control the positioning of a catheter tip in a two-dimensional plane, according to an embodiment.

While various embodiments are amenable to various modifications and alternative forms, specifics thereof have been shown by way of example in the drawings and will be described in detail. It should be understood, however, that the intention is not to limit the claimed subject matter to particular embodiments described. On the contrary, the intention is to cover all modifications, equivalents, and alternatives falling within the scope of the subject matter as defined by the claims.

DETAILED DESCRIPTION OF THE DRAWINGS

Embodiments disclosed herein include mechanisms for actuation and control of a cable-driven end effector. Proposed systems can comprise a variety of features, such as a control member with a control surface, motion stage system (i.e. axis system), and a cable actuation mechanism with at least one connector mechanism such as spring-actuated pistons or a lever. Furthermore, embodiments can also include modifications such as the addition of a gearbox and/or encoders to track cable displacements.

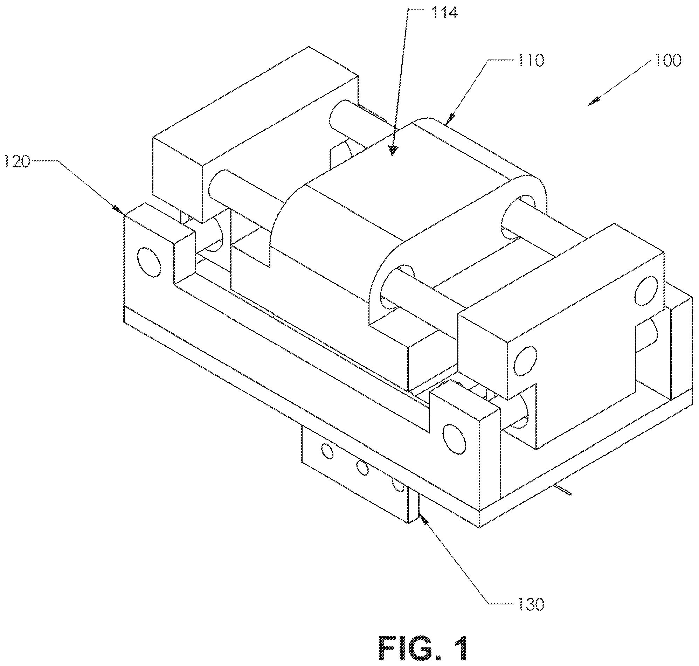

FIGS. 1-5B depict a control mechanism 100, according to an embodiment. Control mechanism 100 may additionally be referred to and understood as a "steering mechanism" at times in this disclosure. Further, control mechanism 100 can also be understood to represent a "cable driven parallel manipulator control mechanism" or "device control mechanism" at times throughout this disclosure and claims that provides passive cable control. Such a cable driven parallel manipulator control mechanism can provide passive cable control in some embodiments. Likewise, a device control mechanism can be understood to control a plurality of cables.

In various embodiments, control mechanism 100 includes a control member 110 having a control surface 112 (in a lower recess) and an input surface 114 (on top) mounted on a motion stage (specifically, a two-axis rail system 120 in FIGS. 1-5B), and a cable actuation mechanism 130 located inferior to the control surface 112. It should be understood that in some embodiments user manipulation of input surface 114, as by urging of a user's thumb, could be used to provide input to the control mechanism 100. Control mechanism 100 can be part of a handle or other user controls in some embodiments.

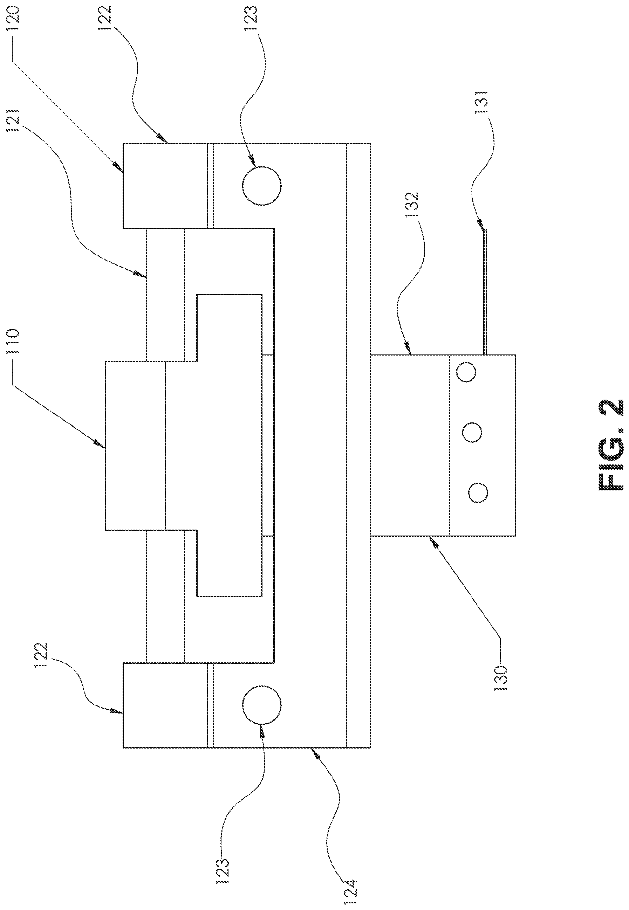

FIG. 2 shows a side view of control mechanism 100, according to an embodiment. As shown, the two-axis rail system 120 consists of two upper shafts 121 mounted on the upper axis mounts 122. Two-axis rail system 120 can be more generally understood to be a two degrees of freedom system on which a control member 110 is mounted and operably slides. Two degrees of freedom systems are not merely limited to two-axis rail systems for purposes of this disclosure The control member 110 slides on the upper shafts 121. The upper axis mounts 122 slide on two lower shafts 123 mounted on the lower axis mounts 124. The cable actuation mechanism 130 is generally located at the base of the control mechanism 100 and is shown to include cables 131 and mounting block 132.

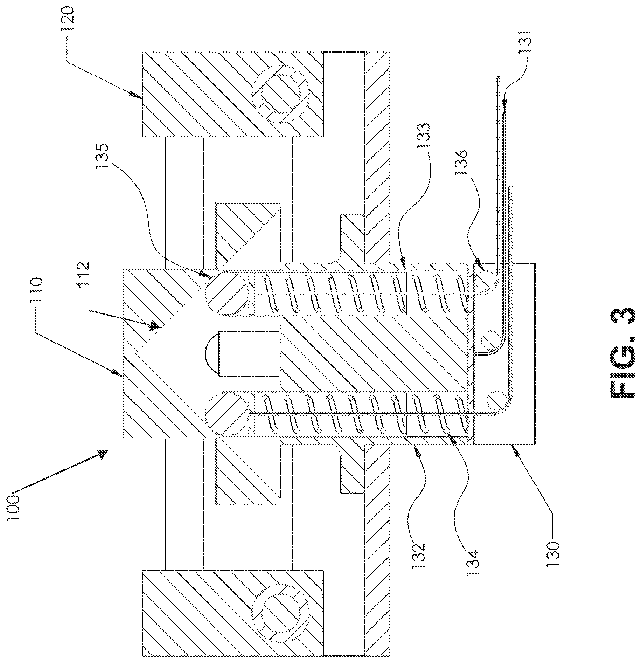

FIG. 3 shows a side cross-section view of control mechanism 100, according to an embodiment. As shown, the cable actuation mechanism 130 is depicted in the cross-section. While not all pistons are viewable in FIG. 3, the cable actuation mechanism 130 should be understood to include four pistons 133, each with a spring 134 and roller 135.

In some embodiments, pistons 133 are not present and instead, some other type of at least one connector mechanism is present. In some embodiments, at least one connector mechanism can be a lever or other mechanism for connecting the cables to the surface. For example, a lever could be used with a spring on one end and which is constrained by the control surface.

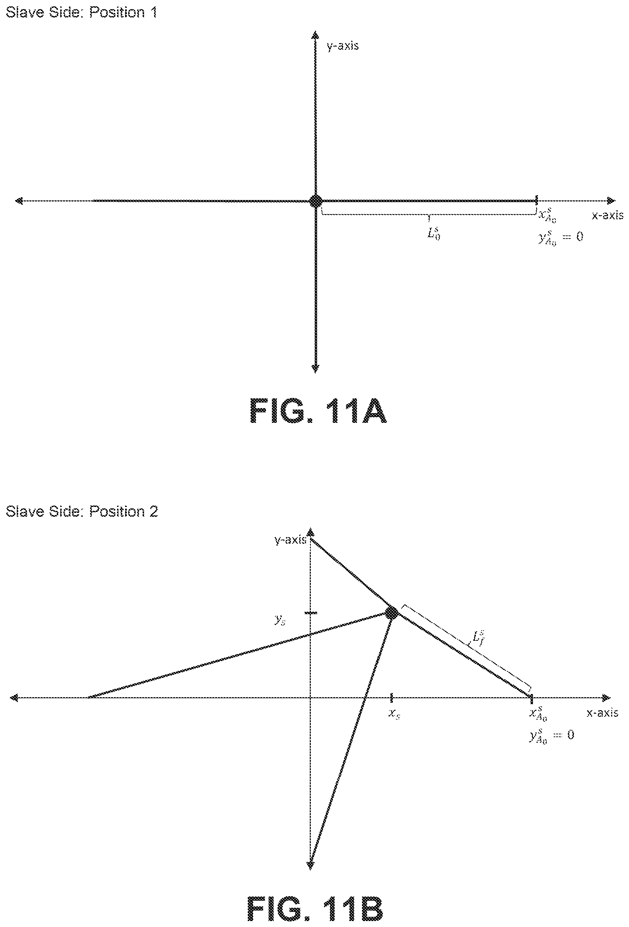

As control member 110 slides on the two-axis rail system 120, springs 134 keep rollers 135 in contact with its control surface 112. Control surface 112 defines a three dimensional profile. In this particular example, the three dimensional profile can be a three-dimensional conical recess and, accordingly, can generally act as a cam for the mechanism. The conical shape of control surface 112 provides a useful structure to ensure necessary cable displacements supplying tension and to communicate proportional adjustments to the device of interest. See FIGS. 11A-D and its discussion related to the conical shape of control surface 112. Other three dimensional profiles are contemplated and possible as well.

Rollers 135 can generally act as cam followers. Rollers 135 are free to rotate to reduce frictional wear on control surface 112. Pistons 133 have a lip to retain rollers 135. As pistons 133 translate in mounting block 132, the cables 131 are actuated by the relevant displacements. Cables 131 pass through cable guides 136 to actuate the end effector (or other feature of the interventional device attached at the end of the cable 131) to the required positions relevant to the embodiment.

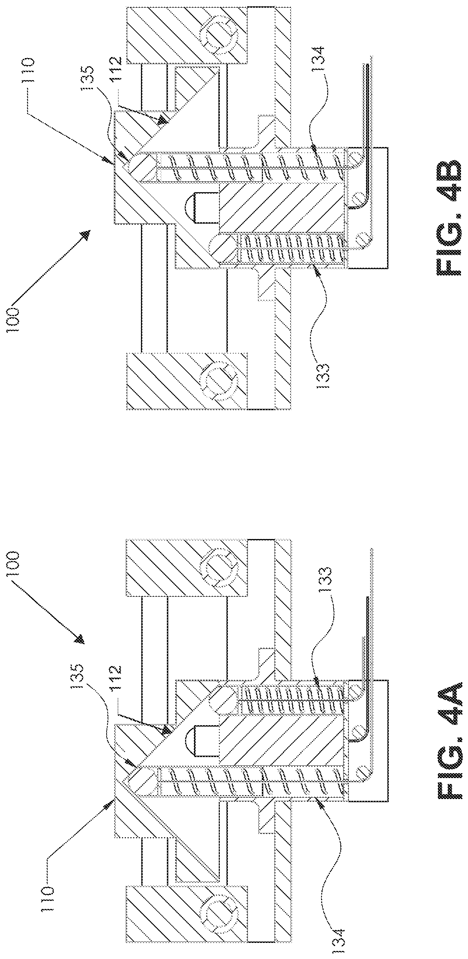

FIGS. 4A and FIG. 4B show side cross-section views of control mechanism 100 with control surface 112 in alternate positions, according to an embodiment. Pistons 133 are shown to have actuated, as springs 134 constrain rollers 135 to contact the control surface 112.

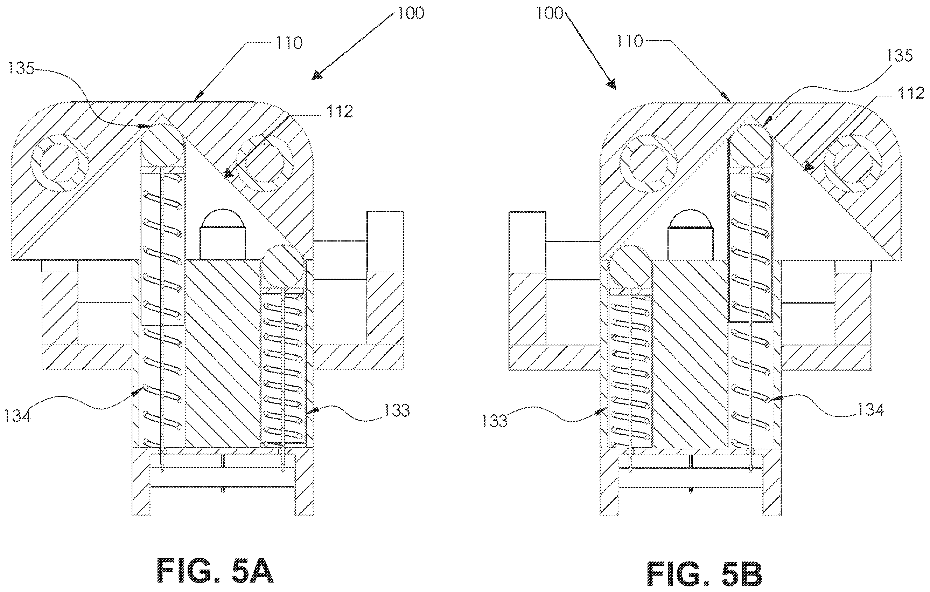

FIGS. 5A and FIG. 5B show an end cross-section view of control mechanism 100 with control surface 112 in alternate positions, according to an embodiment. Pistons 133 are shown to have actuated as springs 134 constrain rollers 135 to contact the control surface 112.

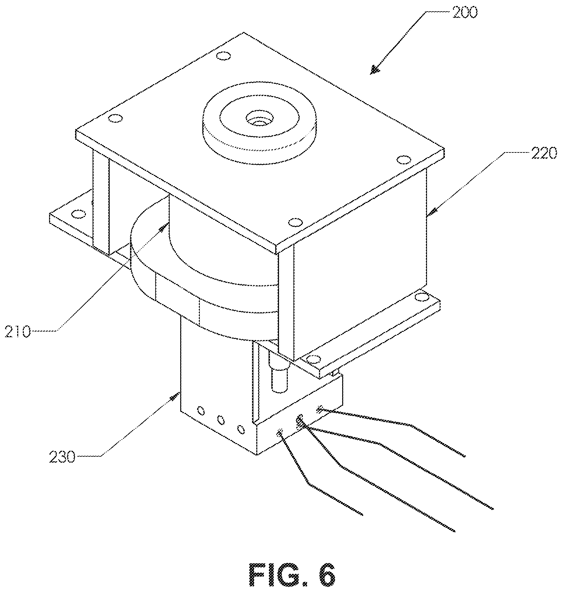

FIGS. 6-10B depict another embodiment of a control surface driven, control mechanism 200. Control mechanism 200 may additionally be referred to and understood as a "steering mechanism" at times in this disclosure. In various embodiments, control mechanism 200 includes a control member 210 having a control surface 212 (in a lower recess) and a joystick 225 (on top) mounted on a motion stage (specifically, a planar roller system 220 in FIGS. 6-10B), and a cable actuation mechanism 230 located inferior to and within the recess defined by control surface 212.

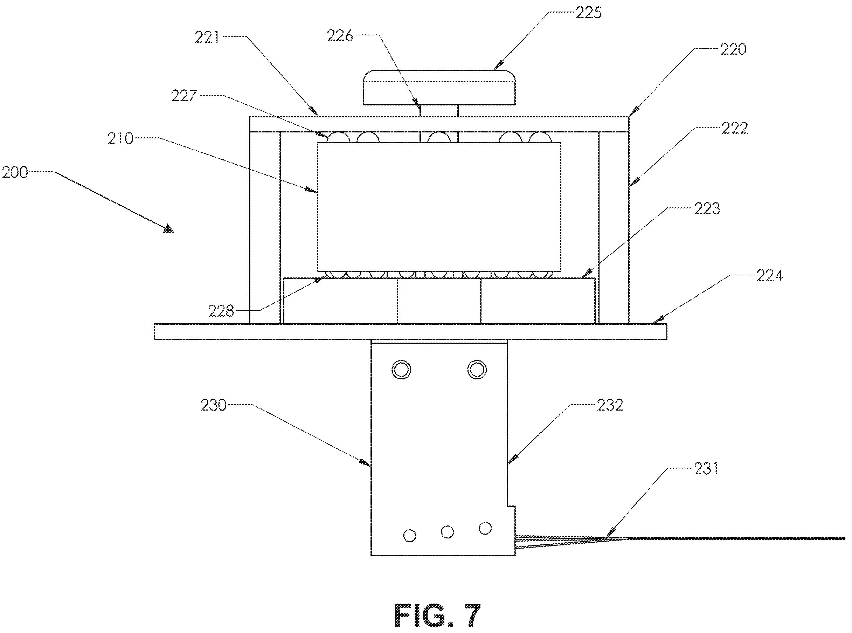

FIG. 7 shows a side view of control mechanism 200, according to an embodiment. In one embodiment, the planar roller system 220 (constituting the motion stage that has two degrees of freedom) consists of a top roller surface 221, mounted with two vertical supports 222 on the mounting plate 224. Other motions stages with two degrees of freedom are possible as well. The control member 210 is sandwiched between the top roller surface 221 and piston block 223, with large rollers 227 and small rollers 228 to distribute the load at the points of contact. The joystick 225 is connected to the control member 210 by joystick mount 226, allowing a user to manually actuate control member 210 and its control surface 212. As the control member 210 is actuated, the large rollers 227 and small rollers 228 roll to reduce the friction. The cable actuation mechanism 230 includes the cables 231 and guide block 232 shown.

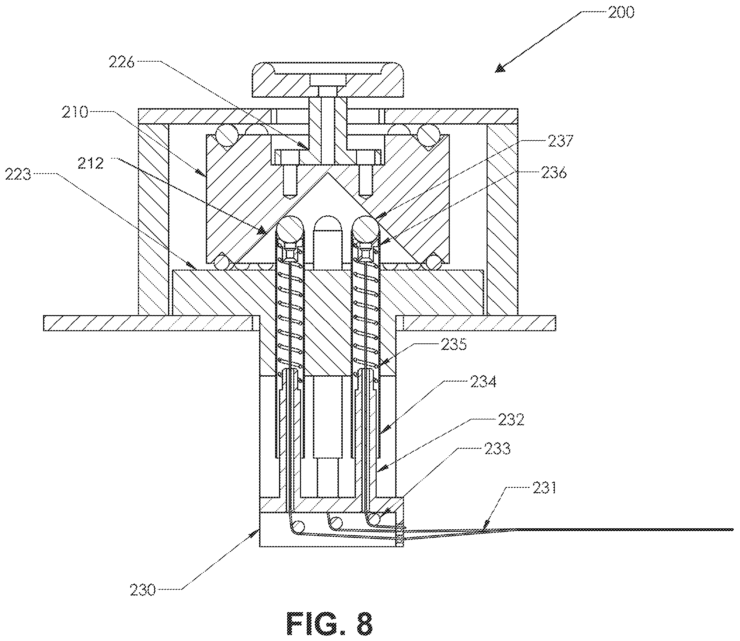

FIG. 8 shows a side cross-section view of control mechanism 200, according to an embodiment. As shown, the cable actuation mechanism 230 is depicted in the cross-section to comprise four pistons 234, each with a spring 235 and roller 237. Specifically, in this embodiment, rollers 237 are balls. As control surface 212 is translated on the planar roller system 220, springs 235 keep the rollers 237 in contact with the control surface 212. Control surface 212 defines a three-dimensional conical recess and accordingly, can generally act as a cam for the mechanism. A conical shape for a control surface 212 can be advantageous and useful for this purpose, as previously described. The cables 231 are fixed within the pistons 234 by the cable mounts 236. The cables 231 travel through the cable guides in the guide block 232 over the guide shafts 233, which reduce friction on the cables 231. As pistons 234 translate in piston block 223, the cables 231 are actuated by the relevant displacements. Cables 231 continue to their termination point to actuate the end effector (or other feature of the interventional device attached at the end of the cable 231) to the required positions relevant to the embodiment.

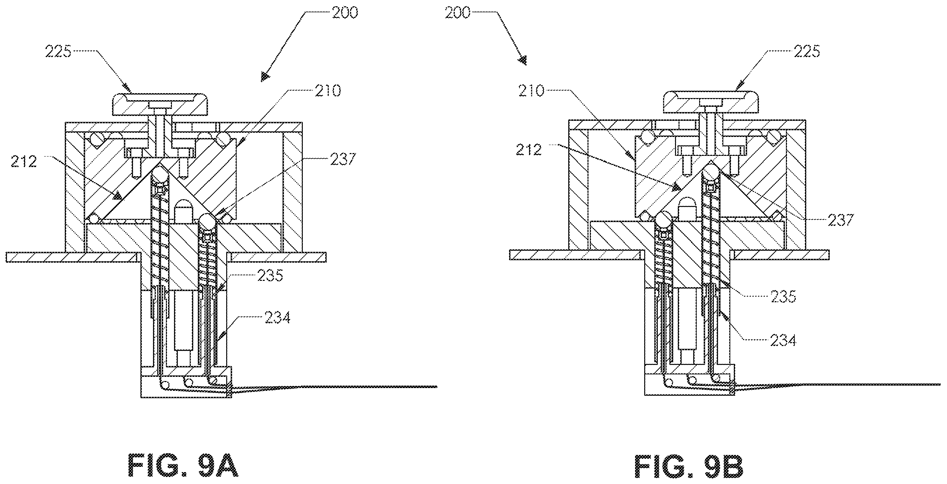

FIG. 9A and FIG. 9B show a side cross-section view of control mechanism 200 with control member 210 and its control surface 212 in alternate positions, according to an embodiment. Pistons 234 are shown to have actuated, as springs 235 constrain rollers 237 to contact the control surface 212.

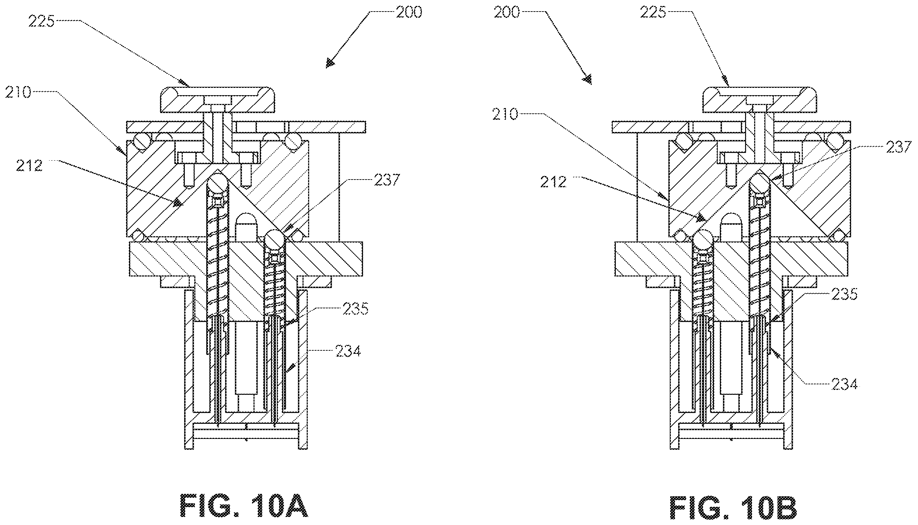

FIG. 10A and FIG. 10B show an end cross-section view of control mechanism 200 with control member 210 and its control surface 212 in alternate positions, according to an embodiment. Pistons 234 are shown to have actuated, as springs 235 constrain rollers 237 to contact the control surface 212.

The following description and FIGS. 11A-D demonstrate the shape for a three-dimensional control surface that can be used to control the positioning of the end effector of a desired device (such as a catheter tip) in a two-dimensional plane, which is shown to be an inverted cone. Moreover, the required geometry of a handle and the relationship between user input and resulting end effector motion is shown to be proportional.

For purposes here, the end effector is manipulated by several cables, each using a single anchor as a leverage point. The control surface needs to change the lengths of the cables on the handle side to match the change of lengths of the cables on the end effector side. Here, the control surface is referred to as the master, and the end effector is referred to as the slave. Their corresponding coordinate systems are indicated by a superscript m, and s respectively.

Let $\alpha$ be the gear ratio between the slave and the master. That is, if a cable is retracted by 1 millimeter in the master side, the gearing system will result in a retraction of $\alpha$ millimeters at the slave side.

$$\alpha \equiv gear\ ratio \quad (Eq.\ 1)$$

The coordinates of the ith anchor at the slave side will be called ( $x_{A_i}^s, y_{A_i}^s$ ) and the coordinates of the corresponding piston in the handle will be ( $x_{P_i}^m, y_{P_i}^m$ ) both of which are assumed constant. This explanation is presented in full generality, and so can be separately applied to any of the several cables (values of i). The figures provided focus on the rightmost cable.

The displacement of the control surface is called ($x_m$, $y_m$) and is considered arbitrary. The resulting displacement of the catheter tip is called ($x_s$, $y_s$) and is a function of the fixed geometry (i.e. the anchor and piston position and the equation of the surface) as well as the current displacement of the surface.

Slave Side (two-dimensional plane):

The change of cable length on the slave side is calculated by taking the initial cable length ($L_0$) at position 1 of the catheter (shown in FIG. 11A) and subtracting it from the final cable length ($L_f$) at position 2 of the catheter (shown in FIG. 11B).

$$\Delta L_i^s = L_f^s - L_0^s \quad (Eq.\ 2)$$

Using Pythagoras' theorem, the initial (FIG. 11A) and final (FIG. 11B) cable lengths for the anchor can be determined. The following equation is in reference to the ith cable where i is arbitrary.

$$\Delta L_i^s = \sqrt{\left(x_{A_i}^s - x_s\right)^2 + \left(y_{A_i}^s - y_s\right)^2} - \sqrt{x_{A_i}^{s\,2} + y_{A_i}^{s\,2}} \quad (Eq.\ 3)$$

Master Side (three-dimensional surface):

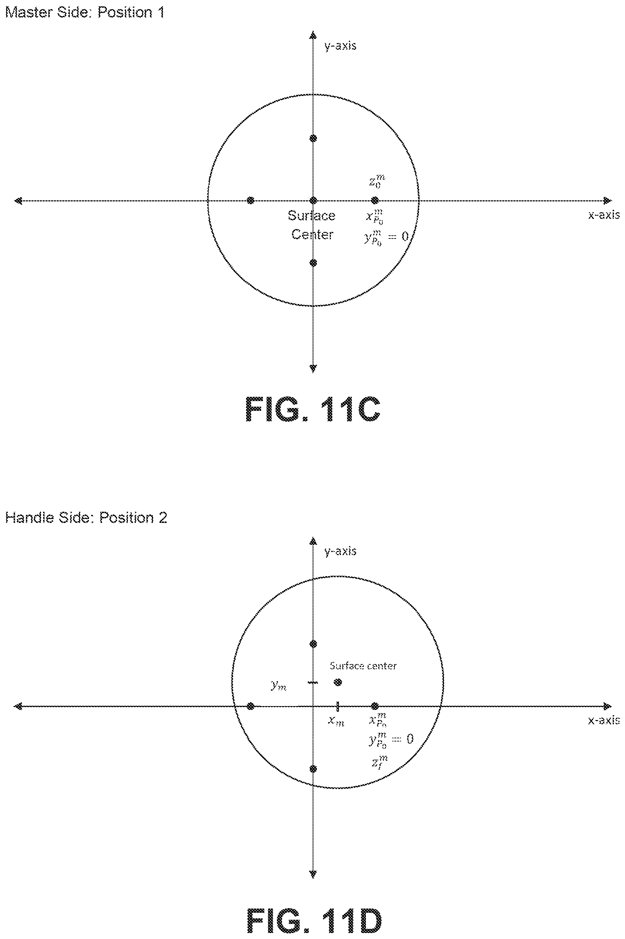

The cables are individually constrained to the surface via the pistons. They are fixed in their x and y positions (like the anchors on the catheter side) so the z position of the cable is determined by the surface's height at that position. The surface is free to move along the x and y axes. The change in cable length is calculated by finding the initial vertical position ($z_0$) of the cable (shown in FIG. 11C) and subtracting it from the final vertical position ($z_f$) of the cable (shown in FIG. 11D)

$$\Delta L_i^m = z_f^m - z_0^m \quad (Eq.\ 4)$$

The initial (FIG. 11A) and final (FIG. 11B) x and y positions for the pistons with respect to the surface center can be determined. The final x and y positions also depend on the displacement of the surface. This allows the positions to be put in terms of the equation of the height of the surface as a function of x and y.

$$\Delta L_i^m = z\left(x_{P_i}^m - x_m, y_{P_i}^m - y_m\right) - z(x_m, y_m) \quad (Eq.\ 5)$$

The relationship between $\Delta L_i^s$ and $\Delta L_i^m$ is due to the conservation of cable length. A negative relation occurs because increase in the length of cable in the handle must result in decrease in the length of cable at the catheter tip. Moreover, due to the gearing, an increase in cable length in the handle of $\alpha$ millimeters is required to cause a decrease in cable length of 1 millimeter at the catheter tip. Thus,

$$\Delta L_i^m = -\frac{1}{\alpha}\Delta L_i^s \quad (Eq.\ 6)$$

$$z\left(x_{P_i}^m - x_m, y_{P_i}^m - y_m\right) - z\left(x_{P_i}^m, y_{P_i}^m\right) =$$

$$-\sqrt{\left(\frac{x_{A_i}^s}{\alpha} - \frac{x_s}{\alpha}\right)^2 + \left(\frac{y_{A_i}^s}{\alpha} - \frac{y_s}{\alpha}\right)^2} + \sqrt{\left(\frac{x_{A_i}^s}{\alpha}\right)^2 + \left(\frac{y_{A_i}^s}{\alpha}\right)^2} \quad (Eq.\ 7)$$

Accordingly, the following equation of the surface is well suited to elegantly matching up the two sides of this equality.

$$z(x,y) = H_0 - \sqrt{x^2 + y^2} \quad \textit{(Eq. 8)}$$

This is the equation of a cone with a slope of 1 as claimed. The height offset, $H_0$, is arbitrary and has no bearing on the result. Thus, any height offset that is convenient may be used.

Moreover, this approach dictates that the geometry of the pistons in the handle should be a scaled-down version of the geometry of the anchors at the catheter tip.

$$\left(x_{P_i}^m, y_{P_i}^m\right) = \frac{1}{\alpha}\left(x_{A_i}^s, y_{A_i}^s\right) \quad \textit{(Eq. 9)}$$

Further, this explicitly describes that the catheter tip displacement is a scaled-up version of the control surface displacement.

$$(x_s, y_s) = \alpha(x_m, y_m) \quad \textit{(Eq. 10)}$$

The slope of the cone may be changed to a number other than 1. For example, changing the slope to 2 would double the displacements of the cables, which can be combined with a gear ratio of one half in the tensioning system to leave the overall output unchanged. This can be useful because it can decrease the required width of the handle and improve ergonomics.

Accordingly, as demonstrated by this explanation and FIGS. 11A-D, using a three-dimensional conical shape for control surface 212 provides a desirable physical structure for the control mechanism and allows for a particularly useful apparatus.

Control mechanisms (such as 100 and 200, for example) have broad applicability to cable driven devices. Moreover, there are many applications for cable-driven master/slave systems. One application can be found in minimally invasive catheter-based procedures. These procedures are typically guided by flexible elongate members that can be manipulated from outside the body with two degrees-of-freedom, which limits the available workspace. A cable-driven end effector can be used in place of this to add extra degrees-of-freedom and allow the operator better control. Moreover, due to the direct mechanical connection, the operator can also receive haptic feedback from the end effector.

Other potentially relevant embodiments and applications of catheters and the like are in forward-looking imaging probes. One of the main limitations of miniaturizing imaging probes is that the limited space available at the tip of a miniaturized device constrains our ability to integrate sensors or transducers that permit a large field of view (FOV). Using a cable-driven catheter that leverages an expandable structure, the small FOV transducers or sensors can be steered to multiple positions to take readings. Position data taken from the sensors can be used to combine the numerous readings into a single, large FOV image. This can be applied to various imaging modalities such as ultrasound and optical coherence topography (OCT).

Another application lies in confined space robotics. In some embodiments, continuum robots can be used to access hard-to-reach areas while allowing for precise, remote control of an end-effector. Possible applications for confined-space robotics include search and rescue, industrial and nuclear maintenance robots, aerospace and automotive manufacturing, and endoscopy.

As previously noted, embodiments of the proposed systems and methods generally incorporate a control surface (e.g., a three-dimensional conical surface) coupled to a two-degrees-of-freedom motion stage to act as a master side for providing input to the system. In an embodiment, one end of each included cable is connected to a piston, which is constrained to contact the control surface (e.g., by utilization of springs). The other end of the cables is coupled to the slave device of interest to be controlled. As the control surface is translated along the two-dimensional plane, the pistons travel perpendicularly to the plane, resulting in cable displacements that actuate the slave end effector, such as the distal tip of a catheter, for example. This system allows the user to directly adjust the position of the control surface, which results in a corresponding proportional adjustment of the catheter tip via the attached cables.

In some embodiments, the cables may connect to a gearing system before transitioning to the slave. As discussed in the preceding, this has the effect of changing the geometry such that a proportionally smaller displacement of the control surface will be required for a given displacement of the end effector. This allows for the footprint of the master control to be adjusted for the application. In another embodiment, the system can be geared to increase the amount of force applied to the end effector of an interventional device. In addition, this may also be used to adjust the resolution between the master and the slave.

Control mechanisms (such as 100 and 200, for example) can be structured such that the dimensional displacement of the control surface is proportional in both direction and magnitude to the displacement of the end effector, for example. Moreover, the position and geometry of the pistons in the master are proportional to the geometry of the cable anchor points of a slave frame. Also, the position and geometry of the center of the cylinder, relative to the pistons in the master, is proportional to the position and geometry of the end effector relative to the expanded anchors on the slave. The proportionality constant is the gear ratio between the master and slave mechanisms.

In some embodiments, there may only be four cables used for the actuation of the device. Typically, in this scenario, the anchor points in the frame of the slave will be in the shape of a square. This allows the maximum possible coverage of the full area with just four anchor points for the cables. Correspondingly, there will then be four pistons moving perpendicular to the conical surface on the master side, arranged in the shape of a square as well. However, the system being proposed can be much more general and allow for an arbitrary number of anchors in arbitrary positions. For example, in some embodiments, anchors can be located at the vertices of regular polygons with an increasing number of sides. There will then be a corresponding number of cables, and the pistons in the master will be arranged in a corresponding polygon. This has the advantage of allowing the end effector to traverse a more significant proportion of the circular cross-sectional area as the number of vertices of the polygon increases.

In an embodiment, position sensors including devices like linear or rotary encoders, can be added in the master to track the position of the control surface or the displacement of the cables. In such embodiments, the sensor data can be used to visualize the position of the slave device or end effector relative to its available workspace and shown to the operator via a user interface.

In one embodiment, the control mechanism (such as 100 or 200, for example) can be applied to a cable-actuated steerable catheter with an expandable frame, such as the steerable catheter configuration disclosed in WO 2019/213215 A1 to Tavallaei et al. The expandable frame can be used as a rigid leverage point for the cables to actuate an end effector, such as the distal tip of a catheter. This allows for a fully mechanical solution to the master side of the catheter. Position sensors (including linear or rotary encoders, etc.) can also be used to track the catheter tip and provide feedback to the user.

Also, in an embodiment, a forward-looking imaging transducer can be mounted to the end effector. In such an embodiment, the end effector can be moved to various positions, and the imaging probe can be used with positioning data to reconstruct a large field of view (FOV) image. This can be applied to a catheter as discussed in the previous embodiment, or to robotics in a confined workspace for search and rescue applications or for imaging systems in hard-to-reach spaces. Various imaging modalities or sensors may be integrated into the system. Examples include but are not limited to optic-based sensors, ultrasound transducers, or radiation sensors. In such an embodiment, linear or rotary actuators or motors can be used to actuate the position of the control surface in the master. This is particularly useful for imaging probes as it will allow the probe to be swept across a cross-section to reconstruct an image autonomously.

In further embodiments, where the use of a motorized design is permissible, the use of the control surface can reduce the number of motors necessary to the two required to control the two-dimensional translation of the control surface.

Also, in an embodiment, the cables can be attached to the control surface at a single point. For example, a different type of design having three cables and a very elongated surface is possible. In this embodiment, the control surface will be a three-dimensional surface that corresponds to the changes in length required for the individual cables in a single attachment point. In such an embodiment, the cables would be constrained to the surface, and as the attachment point for the cables is moved on the three-dimensional surface the length of the cables on the surface would change in proportion such that the slave device would be controlled in the two-dimensional surface of interest. For other embodiments, alternate mounting methods can be used for the cables on either the end effector side or the control side. Various three-dimensional control surfaces can be used to account for the mounting methods, according to these embodiments.

The proposed method can also be used to actuate a continuum cable-driven manipulator that is actuated by a number of cables. For example, a deflectable catheter that is cable-driven and utilizes more than one cable to deflect its distal end can utilize the same strategy. Namely, a cam surface (also referred to at times herein as a "cam guide surface") can be coupled to the cables such that the motion of the cam surface leads to the adjustment in length of the cables. This permits a passive remote actuation method and provides a mapping between the master unit manipulation and the catheter tip motion based on the profile of the cam surface.

Various embodiments of systems, devices, and methods have been described herein. These embodiments are given only by way of example and are not intended to limit the scope of the claimed subject matter. It should be appreciated, moreover, that the various features of the embodiments that have been described may be combined in various ways to produce numerous additional embodiments. Moreover, while various materials, dimensions, shapes, configurations, and locations, etc. have been described for use with disclosed embodiments, others besides those disclosed may be utilized without exceeding the scope of the claimed subject matter.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

**Claims**

1. A cable driven parallel manipulator control mechanism that provides passive cable control, comprising:

   a control member (110, 210) with a control surface (112, 212) defining a three-dimensional profile;
   a motion stage (120, 220), having at least two degrees of freedom, operably coupled to the control member;
   a cable actuation mechanism (130, 230) located inferior to the control surface, including:

   at least one connector mechanism (133, 234) constrained to contact the control surface; and
   a plurality of cables (131, 231), each having a first end and a second end, wherein the first end is connected to one of the at least one connector mechanism and the second end provides coupled attachment to a device being controlled; and
   **characterised in that** translation of the control member along a two dimensional plane causes the at least one connector mechanism to travel perpendicularly to the two dimensional plane resulting in cable displacements that actuate the device being controlled in proportion to translation of the control member.

2. The cable driven parallel manipulator control mechanism of claim 1, wherein the device being controlled is an interventional device.

3. The cable driven parallel manipulator control mechanism of claim 1, wherein the three-dimensional profile is a conical recess.

4. The cable driven parallel manipulator control mechanism of claim 1, wherein the at least one connector includes a plurality of pistons (133, 234), each including a spring (134, 235) and roller (135, 237) constrained to contact the control surface.

5. The cable driven parallel manipulator control mechanism of claim **1,** wherein the at least one connector includes a lever that contains a spring at one end.

6. The cable driven parallel manipulator control mechanism of claim **1,** wherein the motion stage is a two degrees of freedom system on which the control member is mounted and operably slides;
   optionally wherein the two degrees of freedom system includes two upper shafts mounted to two upper axis mounts, wherein the two upper axis mounts slide on two lower shafts that are mounted on two lower axis mounts.

7. The cable driven parallel manipulator control mechanism of claim **1,** wherein the motion stage is a planar roller system on which the control member is mounted and operably slides;
   optionally wherein the planar roller system includes a top roller surface, a plurality of vertical supports, and a mounting plate.

8. The cable driven parallel manipulator control mechanism of claim **1,** wherein the cable actuation mechanism includes a mounting block.

9. The cable driven parallel manipulator control mechanism of claim **1,** further including a gearing system connected to the plurality of cables;
   optionally wherein the gearing system is geared to increase the amount of force applied at the interventional device.

10. The cable driven parallel manipulator control mechanism of claim 1, wherein the cable actuation mechanism includes cable guides (136, 233).

11. The cable driven parallel manipulator control mechanism of claim 1, wherein the cable actuation mechanism includes a piston block.

12. The cable driven parallel manipulator control mechanism of claim 1, wherein a plurality position sensors are included to track at least one of a position of the control surface and a displacement of at least one of the plurality of cables.

13. The cable driven parallel manipulator control mechanism of claim 1, wherein the device being controlled is a cable-actuated steerable catheter with an expandable frame.

14. The cable driven parallel manipulator control mechanism of claim 1, wherein the plurality of cables of the cable actuation mechanism includes more than three cables which are anchored to the cable actuation mechanism in the shape of a polygon.

15. The cable driven parallel manipulator control mechanism of claim 1, wherein the device being controlled includes a forward-looking imaging transducer.


**Patentansprüche**

1. Seilgetriebener paralleler Manipulator-Steuermechanismus, der eine passive Seilsteuerung bereitstellt, umfassend:

ein Steuerelement (110, 210) mit einer Steuerfläche (112, 212), die ein dreidimensionales Profil definiert;
eine Bewegungsstufe (120, 220), die mindestens zwei Freiheitsgrade aufweist, die mit dem Steuerelement wirkgekoppelt ist;
einen Seilbetätigungsmechanismus (130, 230), der sich unterhalb der Steuerfläche befindet, umfassend:

mindestens einen Verbindermechanismus (133, 234), der so eingeschränkt ist, dass er die Steuerfläche berührt; und
mehrere Seile (131, 231), die jeweils ein erstes Ende und ein zweites Ende aufweisen, wobei das erste Ende mit einem des mindestens einen Verbindermechanismus verbunden ist und das zweite Ende eine gekoppelte Befestigung an einer zu steuernden Vorrichtung bereitstellt; und
**dadurch gekennzeichnet, dass**
eine Translation des Steuerelements entlang einer zweidimensionalen Ebene bewirkt, dass sich der mindestens eine Verbindermechanismus senkrecht zur zweidimensionalen Ebene bewegt, was zu Seilverschiebungen führt, die die zu steuernde Vorrichtung proportional zur Translation des Steuerelements betätigen.

2. Seilgetriebener paralleler Manipulator-Steuermechanismus nach Anspruch 1, wobei die zu steuernde Vorrichtung eine Eingriffsvorrichtung ist.

3. Seilgetriebener paralleler Manipulator-Steuermechanismus nach Anspruch 1, wobei das dreidimensionale Profil eine konische Aussparung ist.

4. Seilgetriebener paralleler Manipulator-Steuermechanismus nach Anspruch 1, wobei der mindestens eine Verbinder mehrere Kolben (133, 234) umfasst, die jeweils eine Feder (134, 235) und eine Rolle (135, 237) umfassen, die so eingeschränkt ist, dass sie die Steuerfläche berührt.

5. Seilgetriebener paralleler Manipulator-Steuermechanismus nach Anspruch 1, wobei der mindestens eine Verbinder einen Hebel umfasst, der an einem Ende eine Feder enthält.

6. Seilgetriebener paralleler Manipulator-Steuermechanismus nach Anspruch 1, wobei die Bewegungsstufe ein System mit zwei Freiheitsgraden ist, auf dem das Steuerelement montiert ist und betriebsfähig gleitet;
optional wobei das System mit zwei Freiheitsgraden zwei obere Wellen umfasst, die an zwei oberen Achsenhalterungen montiert sind, wobei die zwei oberen Achsenhalterungen auf zwei unteren Wellen gleiten, die an zwei unteren Achsenhalterungen montiert sind.

7. Seilgetriebener paralleler Manipulator-Steuermechanismus nach Anspruch 1, wobei die Bewegungsstufe ein planares Rollensystem ist, auf dem das Steuerelement montiert ist und betriebsfähig gleitet; optional wobei das planare Rollensystem eine obere Rollenfläche, mehrere vertikale Stützen und eine Montageplatte umfasst.

8. Seilgetriebener paralleler Manipulator-Steuermechanismus nach Anspruch 1, wobei der Seilbetätigungsmechanismus einen Montageblock umfasst.

**9.** Seilgetriebener paralleler Manipulator-Steuermechanismus nach Anspruch 1, der ferner ein Getriebesystem umfasst, das mit den mehreren Seilen verbunden ist;
wobei optional das Getriebesystem dazu ausgelegt ist, die an der Eingriffsvorrichtung aufgebrachte Kraft zu erhöhen.

**10.** Seilgetriebener paralleler Manipulator-Steuermechanismus nach Anspruch 1, wobei der Seilbetätigungsmechanismus Kabelführungen (136, 233) umfasst.

**11.** Seilgetriebener paralleler Manipulator-Steuermechanismus nach Anspruch 1, wobei der Seilbetätigungsmechanismus einen Kolbenblock umfasst.

**12.** Seilgetriebener paralleler Manipulator-Steuermechanismus nach Anspruch 1, wobei mehrere Positionssensoren enthalten sind, um eine Position der Steuerfläche und/oder eine Verschiebung mindestens eines der mehreren Seile nachzuverfolgen.

**13.** Seilgetriebener paralleler Manipulator-Steuermechanismus nach Anspruch 1, wobei die zu steuernde Vorrichtung ein seilbetätigter lenkbarer Katheter mit einem expandierbaren Rahmen ist.

**14.** Seilgetriebener paralleler Manipulator-Steuermechanismus nach Anspruch 1, wobei die mehreren Seile des Seilbetätigungsmechanismus mehr als drei Seile umfassen, die in der Form eines Polygons an dem Seilbetätigungsmechanismus verankert sind.

**15.** Seilgetriebener paralleler Manipulator-Steuermechanismus nach Anspruch 1, wobei die zu steuernde Vorrichtung einen nach vorne gerichteten Bildwandler umfasst.

**Revendications**

**1.** Mécanisme de commande de manipulateur parallèle entraîné par câbles qui assure une commande de câble passive, comprenant :

un élément de commande (110, 210) présentant une surface de commande (112, 212) définissant un profil tridimensionnel ;
un étage de mouvement (120, 220), présentant au moins deux degrés de liberté, accouplé fonctionnellement à l'élément de commande ;
un mécanisme d'actionnement de câble (130, 230) situé plus bas que la surface de commande, comprenant :

au moins un mécanisme connecteur (133, 234) contraint à entrer en contact avec la surface de commande ; et
une pluralité de câbles (131, 231), ayant chacun une première extrémité et une seconde extrémité, la première extrémité étant reliée à l'un de l'au moins un mécanisme connecteur et la seconde extrémité fournissant une fixation accouplée à un dispositif commandé ; et
**caractérisé en ce que**
la translation de l'élément de commande le long d'un plan bidimensionnel amène l'au moins un mécanisme connecteur à se déplacer perpendiculairement au plan bidimensionnel, ce qui entraîne des déplacements de câble qui actionnent le dispositif commandé proportionnellement à la translation de l'élément de commande.

**2.** Mécanisme de commande de manipulateur parallèle entraîné par câbles selon la revendication 1, le dispositif commandé étant un dispositif d'intervention.

**3.** Mécanisme de commande de manipulateur parallèle entraîné par câbles selon la revendication 1, le profil tridimensionnel étant un évidement conique.

**4.** Mécanisme de commande de manipulateur parallèle entraîné par câbles selon la revendication 1, l'au moins un connecteur comprenant une pluralité de pistons (133, 234), comportant chacun un ressort (134, 235) et un rouleau (135, 237) contraint à entrer en contact avec la surface de commande.

**5.** Mécanisme de commande de manipulateur parallèle entraîné par câbles selon la revendication 1, l'au moins un connecteur comprenant un levier qui contient un ressort à une extrémité.

**6.** Mécanisme de commande de manipulateur parallèle entraîné par câbles selon la revendication 1, l'étage de mouvement étant un système à deux degrés de liberté sur lequel l'élément de commande est monté et coulisse de manière opérationnelle ;
éventuellement, le système à deux degrés de liberté comprenant deux arbres supérieurs montés sur deux supports d'axe supérieur, les deux supports d'axe supérieur coulissant sur deux arbres inférieurs qui sont montés sur deux supports d'axe inférieur.

**7.** Mécanisme de commande de manipulateur parallèle entraîné par câbles selon la revendication 1, l'étage de mouvement étant un système de rouleaux plans sur lequel l'élément de commande est monté et coulisse de manière opérationnelle ;
éventuellement, le système de rouleaux plans comprenant une surface de rouleaux supérieure, une pluralité de supports verticaux et une plaque de montage.

**8.** Mécanisme de commande de manipulateur parallèle entraîné par câbles selon la revendication 1, le mécanisme d'actionnement de câble comprenant un bloc de montage.

**9.** Mécanisme de commande de manipulateur parallèle entraîné par câbles selon la revendication 1, comprenant en outre un système d'engrenage relié à la pluralité de câbles ;
éventuellement, le système d'engrenage étant engrené pour augmenter la quantité de force appliquée au niveau du dispositif d'intervention.

**10.** Mécanisme de commande de manipulateur parallèle entraîné par câbles selon la revendication 1, le mécanisme d'actionnement de câble comprenant des guides de câble (136, 233).

**11.** Mécanisme de commande de manipulateur parallèle entraîné par câbles selon la revendication 1, le mécanisme d'actionnement de câble comprenant un bloc de piston.

**12.** Mécanisme de commande de manipulateur parallèle entraîné par câbles selon la revendication 1, une pluralité de capteurs de position étant inclus pour suivre au moins l'un d'une position de la surface de commande et d'un déplacement d'au moins l'un de la pluralité de câbles.

**13.** Mécanisme de commande de manipulateur parallèle entraîné par câbles selon la revendication 1, le dispositif commandé étant un cathéter orientable actionné par câbles doté d'un cadre extensible.

**14.** Mécanisme de commande de manipulateur parallèle entraîné par câbles selon la revendication 1, la pluralité de câbles du mécanisme d'actionnement de câble comprenant plus de trois câbles qui sont ancrés au mécanisme d'actionnement de câble sous la forme d'un polygone.

**15.** Mécanisme de commande de manipulateur parallèle commandé par câbles selon la revendication 1, le dispositif commandé comprenant un transducteur d'imagerie orienté vers l'avant.

FIG. 1

FIG. 2

EP 4 259 387 B1

FIG. 3

FIG. 4B

FIG. 4A

FIG. 5B

FIG. 5A

200

220

210

230

FIG. 6

FIG. 7

FIG. 8

FIG. 9A

FIG. 9B

**FIG. 10A**

**FIG. 10B**

Slave Side: Position 1

**FIG. 11A**

Slave Side: Position 2

**FIG. 11B**

Master Side: Position 1

y-axis

$z_0^m$

Surface
Center
$x_{P_0}^m$

$y_{P_0}^m = 0$

x-axis

**FIG. 11C**

Handle Side: Position 2

y-axis

Surface center

$y_m$

$x_m$   $x_{P_0}^m$

$y_{P_0}^m = 0$

$z_f^m$

x-axis

**FIG. 11D**

**EP 4 259 387 B1**

**Patent documents cited in the description**

- US 63123981
- US 2020170701 A1
- US 2010179540 A1
- EP 3653105 A1
- EP 4173576 A1
- WO 2019213215 A1, Tavallaei